# EUROPÄISCHE PATENTSCHRIFT

(11) **EP 3 397 225 B1**
(45) Veröffentlichungstag und Bekanntmachung des Hinweises auf die Patenterteilung: **24.11.2021**
(21) Anmeldenummer: 16819315.9
(22) Anmeldetag: 20.12.2016
(51) Int. Cl.: A61F 13/49, A61F 13/551

(54) **INKONTINENZWEGWERFWINDEL**
INCONTINENCE DIAPER
COUCHE JETABLE POUR INCONTINENCE

(30) Priorität: 29.12.2015 DE 102015226814
(43) Veröffentlichungstag der Anmeldung: 07.11.2018
(73) Patentinhaber: Paul Hartmann AG, 89522 Heidenheim (DE)
(72) Erfinder: OSTERTAG, Wolfgang, 89547 Gerstetten (DE); EBERT, Anselm, 97204 Höchberg (DE); KESSELMEIER, Ruediger, 89233 Burlafingen (DE); DRUMEVA-EBERIUS, Albena, 89129 Langenau (DE)
(74) Vertreter: DREISS Patentanwälte PartG mbB
(86) Internationale Anmeldenummer: PCT/EP2016/082005
(87) Internationale Veröffentlichungsnummer: WO 2017/114695

(56) Entgegenhaltungen:
- WO-A1-2007/035903
- WO-A1-2007/070023
- WO-A1-2007/149017
- WO-A1-2011/106648
- DE-A1-102009 022 529
- US-A1- 2007 246 152

## Beschreibung

Die Erfindung betrifft eine Inkontinenzwegwerfwindel, mit einem einen Saugkörper aufweisenden Hauptteil mit einer Längsrichtung und einer Querrichtung, umfassend einen Vorderbereich mit vorderen seitlichen Längsrändern, einen Rückenbereich mit hinteren seitlichen Längsrändern und einen dazwischen angeordneten, zwischen den Beinen eines Benutzers zu liegen kommenden Schrittbereich, und mit beidseits an den Rückenbereich angefügten hinteren Seitenabschnitten, welche sich in der Querrichtung der Inkontinenzwegwerfwindel über die hinteren seitlichen Längsränder des Hauptteils hinaus erstrecken und im Bereich ihres in Querrichtung freien Endes jeweils wenigstens ein Verschlussmittel tragen, wohingegen an den Vorderbereich keine Seitenabschnitte angefügt sind, sondern die vorderen seitlichen Längsränder des Hauptteils einen frei endenden Längsrand der Windel bilden, wobei die hinteren Seitenabschnitte zum Anlegen und Schließen der Inkontinenzwegwerfwindel an einen Benutzer jeweils entlang einer Umfangsrichtung um den Körper des Benutzers herumlegbar und in überlappende Anordnung mit einer Außenseite des Vorderbereichs bringbar sind, an der sie dann über das jeweilige Verschlussmittel jeweils lösbar anhaftbar sind, wobei die hinteren Seitenabschnitte in eben ausgebreitetem jedoch nicht gedehnten Zustand eine Erstreckung (Q) in der Querrichtung über den jeweiligen hinteren seitlichen Längsrand hinaus aufweisen und innerhalb dieser Erstreckung (Q) in der Querrichtung elastisch dehnbar sind und hierfür einen in der Querrichtung und in der Längsrichtung erstreckten elastischen oder elastifizierten Bereich aufweisen, wobei diese Erstreckung (Q) der hinteren Seitenabschnitte in der Querrichtung eine an den hinteren seitlichen Längsrand anschließende proximale Hälfte und eine frei endende distale Hälfte umfasst.

Bei einer derartigen Inkontinenzwegwerfwindel handelt es sich um eine so genannte T-Form-Windel, die sich von anderen Windelkonzepten grundlegend unterscheidet. Bei derartigen T-Form-Windeln, wie sie beispielsweise in WO 2007/035903 A1 beschrieben sind, sind nur im Rückenbereich Seitenabschnitte an den Hauptteil angefügt, während die Windel im Vorderbereich keine zusätzlichen angefügten Seitenabschnitte aufweist sondern durch jeweilige seitliche Längsränder des Hauptteils begrenzt ist. Die hinteren Seitenabschnitte sind in der Längsrichtung der Windel zumeist ausladend, jedoch in der Regel kürzer als die Erstreckung der hinteren seitlichen Längsränder des Hauptteils im Rückenbereich. In der Querrichtung sind die hinteren Seitenabschnitte von T-Form-Windeln derart erstreckt, dass sie in Überlappung mit der Außenseite des Vorderbereichs des Hauptteils gebracht werden können, damit die im Bereich der jeweiligen freien Enden der hinteren Seitenabschnitte vorgesehenen Verschlussmittel auf die Außenseite des Hauptteils der Windel geschlossen werden können.

Im Unterschied hierzu sind sogenannte Gürtelwindeln in großem Umfang bekannt geworden, bei denen sich beidseits vom Rückenbereich des Windelhauptteils in Querrichtung sehr lange Gürtelabschnitte weg erstrecken, die derart bemessen sind, dass sie um den gesamten Bauchumfang des Benutzers herum auf sich selbst geschlossen werden können. Bei Anlegen einer Gürtelwindel wird das Produkt von hinten gegen den Hüft- oder Rückenbereich des Benutzers gelegt, und es werden sodann die beiden Gürtelabschnitte auf der Bauchseite des Benutzers direkt aufeinander geschlossen. Hierbei werden keinerlei Schrägzugkräfte auf den Gürtel oder den Hauptteil eingeleitet, sondern es wird eine lediglich in Hüftumfangsrichtung wirkende Kraft auf die beiden Gürtelabschnitte ausgeübt. Danach wird der Windelhauptteil zwischen den Beinen des Benutzers hervorgeholt und mittels weiterer Verschlusselemente zumeist gegen die Außenseite des zuvor geschlossenen Gürtels positioniert und fixiert. Es wurden auch bereits Bereiche der Gürtelabschnitte elastisch ausgebildet, z.B. EP 2 029 079 B1.

Bei hier in Rede stehenden T-Form-Windeln, bei denen die hinteren Seitenabschnitte auf die Außenseite des Vorderbereichs des Windelhauptteils geschlossen werden, werden beim Schließen häufig starke Zugkräfte in die Seitenabschnitte und in den Windelhauptteil eingeleitet, weil der Benutzer oder eine Pflegeperson beim Anlegen der Windel bestrebt ist, eine deutliche Überlappungssituation zwischen den hinteren Seitenabschnitten und dem Vorderbereich des Hauptteils herzustellen und hierbei gleichzeitig die für einen Festsitz der Windel erforderliche Zugkraft in das Gesamtsystem einzubringen, so dass die Windel auch dauerhaft am Benutzer gehalten wird. Hierbei kommt es in der Pflegesituation auch zur Ausübung von Schrägzugkräften, also von Zugkräften, die neben einer Komponente in Querrichtung eine Komponente in Längsrichtung aufweisen und im Fügebereich der hinteren Seitenabschnitte an den Hauptteil zu kritischen Zuständen führen. Es kommt dort häufig zum Einreißen der Seitenabschnitte oder zum Auftrennen der Fügeverbindung. Dieses Problem stellt sich bei Gürtelwindeln und auch bei Windeln mit hinten und vorn angefügten Seitenabschnitten in bedeutend geringerem Umfang und Ausmaß. Bei Gürtelwindeln ist die Kinematik des Anlegens eine völlig andere, und bei Windeln mit hinteren und vorderen Seitenabschnitten wird eine typische Anlegesituation bereits durch das Überlappen der jeweiligen hinteren und vorderen Seitenabschnitte hergestellt. WO2007070023 beschreibt absorbierende Wegwerfartikel, z.B. offene Windeln mit an den Rückenbereich angefügten Seitenabschnittsteilen die dehnbare proximale und nicht dehnbare distale Bereiche aufweisen.

US2007246152 beschreibt Inkontinenzwindeln mit an den Rückenbereich angefügten Seitenabschnitten mit proximalen dehnbaren und distalen nicht dehnbaren Bereichen.

Der vorliegenden Erfindung liegt die Aufgabe zu Grunde, eine T-förmige Inkontinenzwegwerfwindel der eingangs genannten Art dahingehend zu verbessern, dass beim Anlegen der Windel an den Körper des Benutzers auftretende Zugkräfte, insbesondere Schrägzugkräfte in geringerem Maße zu Beschädigungen der Windelmaterialien führen, wobei gleichwohl eine kostengünstige Herstellbarkeit des Artikels realisiert werden soll.

Diese Aufgabe wird bei einer Inkontinenzwegwerfwindel der genannten Art erfindungsgemäß dadurch gelöst, dass der jeweilige elastische oder elastifizierte Bereich der hinteren Seitenabschnitte vollständig innerhalb der proximalen Hälfte angeordnet ist und an den zugeordneten hinteren seitlichen Längsrand heranreicht oder einen Abstand von dem hinteren seitlichen Längsrand in der Querrichtung von höchstens 30 mm aufweist, und dass die jeweiligen hinteren Seitenabschnitte in der gesamten distalen Hälfte im wesentlichen undehnbar ausgebildet sind.

Mit der vorliegenden Erfindung wurde erkannt, dass elastisch dehnbare Bereiche bei den hinteren Seitenabschnitten zu keiner Verbesserung beim Anlegen der Windel beitragen, wenn sie in der Querrichtung allzu weit entfernt von dem Rückenbereich des Windelhauptteils angeordnet sind. Sie führen im Gegenteil dazu, dass der Benutzer oder eine Pflegeperson sich genötigt fühlt, eine noch größere Zugkraft und Dehnung aufzuwenden. Es wurde festgestellt, dass auf dehnbare, insbesondere elastisch dehnbare Bereiche in der jeweiligen distalen Hälfte der hinteren Seitenabschnitte ersatzlos verzichtet werden kann, was die Kosten der Herstellung infolge der Reduzierung der zumeist teuren elastischen oder elastifizierten Materialien reduziert.

Wenn in der vorliegenden Anmeldung von den Eigenschaften "elastisch dehnbar", "dehnbar" oder "undehnbar" die Rede ist, so ist die jeweilige Eigenschaft stets in der Querrichtung der Inkontinenzwegwerfwindel gemeint. Im Hinblick auf eine quantifizierbare Abgrenzung elastisch dehnbarer Bereiche von undehnbaren oder nicht elastisch dehnbaren Bereichen wird auf die weiter unten beschriebenen Mess- oder Testmethoden verwiesen.

Es erweist sich weiter als vorteilhaft, wenn der jeweilige elastische oder elastifizierte Bereich der hinteren Seitenabschnitte an den zugeordneten hinteren seitlichen Längsrand heranreicht, jedoch ohne den hinteren seitlichen Längsrand zu überlappen. In Weiterbildung dieses Erfindungsgedankens beträgt der Abstand eines jeweiligen elastischen oder elastifizierten Bereichs der hinteren Seitenabschnitte von dem hinteren seitlichen Längsrand in der Querrichtung höchstens 20 mm, insbesondere höchstens 10 mm. Dies eröffnet die Möglichkeit, dass im unmittelbaren Übergang des außerhalb des Hauptteils liegenden Bereichs des Seitenabschnitts zu einem mit dem Hauptteil überlappenden Bereich des Seitenabschnitts keine dehnbaren Seitenabschnittmaterialien vorgesehen sind, was die Stabilität der Fügeverbindung erhöht.

Daher erweist es sich auch als vorteilhaft, wenn die beiden hinteren Seitenabschnitte mit einem undehnbaren den Hauptteil überlappenden Bereich an den Hauptteil unlösbar angefügt sind.

Es wird weiter vorgeschlagen, die Seitenabschnitte so auszubilden, dass bei Einleitung üblicher die Gebrauchssituation simulierender Kräfte in der Querrichtung der jeweilige elastische oder elastifizierte Bereich in der Querrichtung um wenigstens 70 %, insbesondere um wenigstens 80 %, weiter insbesondere um wenigstens 90 % dehnbar ist. Hierbei ist eine Dehnbarkeit gemeint, die nicht einhergeht mit einer dauerhaften signifikanten plastischen Verformung oder gar Materialschädigung sondern eine elastische Zurückstellung der gedehnten Seitenabschnitte zulässt und bewirkt. Die Werte werden durch entsprechende Anwendung der weiter unten beschriebenen Mess- oder Testmethoden bestimmt.

Die hinteren Seitenabschnitte sind im einfachsten und bevorzugten Fall rechteckförmig ausgebildet, d.h. sie werden durch in der Querrichtung und in der Längsrichtung der Inkontinenzwegwerfwindel verlaufende Ränder begrenzt. Vorzugsweise wird zumindest die proximale Hälfte der Erstreckung (Q) der Seitenabschnitte von in der Querrichtung verlaufenden Rändern begrenzt.

Vorteilhaft ist auch ein jeweiliger elastischer oder elastifizierter Bereich rechteckfömig ausgebildet, das heißt sie werden durch in der Querrichtung und in der Längsrichtung der Inkontinenzwegwerfwindel verlaufende Ränder begrenzt. Weiter vorzugsweise erstreckt sich ein jeweiliger elastischer oder elastifizierter Bereich über die volle Länge der Seitenabschnitte (in der Längsrichtung der Inkontinenzwegwerfwindel).

Die Erstreckung eines jeweiligen elastischen oder elastifizierten Bereichs in der Querrichtung beträgt ungedehnt vorzugsweise 40 bis 120 mm, insbesondere 60 - 100 mm.

Die Erstreckung (Q) der eben ausgebreiteten jedoch nicht gedehnten hinteren Seitenabschnitte in der Querrichtung der Inkontinenzwegwerfwindel über den hinteren seitlichen Längsrand des Hauptteils hinaus beträgt vorzugsweise 130 bis 280 mm, insbesondere 170 bis 250 mm.

Die Erstreckung (B) der hinteren Seitenabschnitte in der Längsrichtung beträgt im Bereich der Anfügung an den Hauptteil 100 bis 200 mm, insbesondere 120 bis 170 mm.

Vorzugsweise ist eine Erstreckung (QE) eines jeweiligen elastischen oder elastifizierten Bereichs in der Querrichtung und eine maximale Erstreckung (Q) der hinteren Seitenabschnitte über den jeweiligen hinteren seitlichen Längsrand hinaus derart bemessen ist, dass das Verhältnis der Erstreckungen (QE/Q) zueinander 0,20 < QE/Q < 0,50, insbesondere 0,30 < QE/Q < 0,45 beträgt.

Es hat sich bei der erfindungsgemäßen T-förmigen Inkontinenzwegwerfwindel als besonders vorteilhaft erwiesen, wenn die Erstreckung (Q) der hinteren Seitenabschnitte in der Querrichtung über den jeweiligen hinteren seitlichen Längsrand hinaus und eine maximale Erstreckung (B) der hinteren Seitenabschnitte in der Längsrichtung derart bemessen ist, dass das Verhältnis der Erstreckungen (Q/B) zueinander 1,0 < Q/B < 2,0 beträgt. Dieses Verhältnis ist bei Gürtelwindeln um ein Vielfaches größer.

Es wird weiter vorgeschlagen, dass die beiden hinteren Seitenabschnitte in der Längsrichtung einen Abstand zu einem hinteren Querrand des Hauptteils von wenigstens 1 mm, insbesondere von wenigstens 5 mm, insbesondere von wenigstens 10 mm, insbesondere von wenigstens 15 mm, insbesondere von höchstens 50 mm aufweisen. Hierdurch ist gewährleistet, dass die beim Anlegen über die Verschlussmittel ausgeübten und dabei in den Rückbereich des Hauptteils eingeleiteten Querzugkräfte auf einen größeren Abschnitt des Hauptteils "verteilt" werden.

Weiter erweist sich als vorteilhaft, wenn eine in der Querrichtung erstreckte und das jeweilige Verschlussmittel auf der schrittzugewandten Seite tangierende gerade Linie den Saugkörper schneidet. Dies kann vorzugsweise dann realisiert werden, wenn die beiden hinteren Seitenabschnitte wie vorstehend erwähnt einen Abstand in der Längsrichtung zum hinteren Querrand des Hauptteils aufweisen. Insbesondere ist vorgesehen, dass eine in der Querrichtung erstreckte und die Seitenabschnitte in der Längsrichtung, im Bereich der Anfügung an den Hauptteil halbierende gerade Linie den Saugkörper schneidet. Es stabilisiert die Anlage des Saugkörpers und unterstützt einen korrekten Sitz der Windel.

Es erweist sich weiter als vorteilhaft, wenn jeder hintere Seitenabschnitt genau ein Verschlussmittel aufweist. Es handelt sich bei den Verschlussmitteln typischerweise um eine Lasche aus einem ein- oder mehrschichtigen Flachmaterial, welches ausgehend von einer in der Regel um einen distalen Längsrand des betrachteten Seitenabschnitts nach innen auf den Seitenabschnitt eingefalteten Konfiguration in eine nach außen ausgefaltete Betriebsstellung entfaltbar ist. In an sich bekannter und daher nicht näher zu beschreibender Weise ist ein jeweiliges Verschlussmittel mit klebenden und/oder mechanisch haftenden Bereichen, Schichten oder Elementen ausgestattet, wie zum Beispiel Haken/Schlaufen-Materialien. Sofern der Seitenabschnitt genau ein Verschlussmittel aufweist, so erweist es sich als vorteilhaft, wenn dieses Verschlussmittel in der Längsrichtung ungefähr mittig in einem distalen Bereich des Seitenabschnitts vorgesehen ist. Weiter erweist es sich als vorteilhaft, wenn das betreffende Verschlussmittel eine Erstreckung in der Längsrichtung zwischen 25 % und 75 % der Erstreckung B des Seitenabschnitts in der Längsrichtung beträgt. Ferner sind die jeweiligen Verschlussmittel vorzugsweise im ein- und ausgefalteten Zustand rechteckförmig ausgebildet. Sie sind in der herstellerseitigen nicht aktiven Konfiguration vorzugsweise nach innen auf sich selbst eingefaltet.

Hinsichtlich der Abmessungen des Hauptteils der Inkontinenzwegwerfwindel hat es sich als vorteilhaft erwiesen, wenn die Erstreckung des Hauptteils in der Querrichtung im Rückenbereich und/oder im Vorderbereich 250 bis 550 mm, insbesondere 300 bis 520 beträgt. Vorzugsweise weisen der Vorderbereich und der Rückenbereiche des Hauptteils dieselbe Quererstreckung (gemessen in mm) auf.

Die Erstreckung des Hauptteils in der Längsrichtung beträgt vorzugsweise 700 bis 1200 mm, insbesondere 800 bis 1100 mm. Der Hauptteil kann im Schrittbereich mit einer Verengung in Querrichtung mithin mit einer Beinöffnungskontur versehen sein. In einer alternativen Ausführungsform ist der Hauptteil rechteckförmig ausgebildet.

Die elastischen oder elastifizierten Bereiche können durch dem Fachmann an sich geläufige Mittel realisiert werden. So können beispielsweise Abschnitte per se elastischer Materialien, wie elastische Folien oder elastische Vliesstoffe, durch Fügeverfahren wie Kleben oder Thermoschweißen oder Ultraschallschweißen, an undehnbare Abschnitte der Seitenabschnitte angefügt werden.

Eine weitere Möglichkeit, eine bereichsweise Elastifizierung zu erreichen, besteht darin, dass die Seitenabschnitte bereichsweise vorzugsweise durch eine als "ring rolling" bekannt gewordene Technologie "aktiviert" werden. Diese Technologie ist beispielsweise in EP 0 650 714 A1 beschrieben. Durch "ring rolling" wird ein an sich nicht dehnbares Material, beispielsweise ein Vlies/Folien-Laminat durch übermäßige Auslenkung zwischen gegeneinander kämmenden Walzen überdehnt. In diesem überdehnten Zustand bringt das zuvor an sich nicht dehnbare Material des Laminates einer Längung im Wesentlichen keinen Widerstand entgegen. Durch Kombination mit einem elastisch dehnbaren Element innerhalb eines derartigen Laminates kann somit eine elastische Dehnbarkeit in dem entsprechend behandelten Bereich erreicht werden. Alternativ hierzu können bereichsweise elastifizierende Mittel wie elastische Folienabschnitte oder Fäden, insbesondere Lycra- oder Spandex-Fäden im vorgespannten Zustand mit den die Seitenabschnitte im Übrigen bildenden Flachmaterialien, insbesondere Vliesstoffmaterialien verbunden werden (stretch-bonding) .

In der Gebrauchssituation werden die hinteren Seitenabschnitte in Überlappung mit der Außenseite des Vorderbereichs des Hauptteils gebracht, damit die im Bereich der jeweiligen freien Enden an beiden hinteren Seitenabschnitten vorgesehenen Verschlussmittel auf die Außenseite des Hauptteils der Windel geschlossen werden können. Hierzu sind die Verschlussmittel und mindestens ein Bereich der Außenseite des Hauptteils als Verschlusssystem ausgebildet. Insbesondere weisen die Verschlussmittel hierzu mechanische Verschlusselemente wie Kletthakenelemente auf, insbesondere auch in Kombination mit haftklebenden Bereichen, vermittels derer die Verschlussmittel lösbar haftend mit der Außenseite des Hauptteils in Eingriff bringbar sind. Hierzu hat es sich als vorteilhaft erwiesen, wenn die Außenseite des Hauptteils zumindest bereichsweise, vorzugsweise vollständig durch einen entsprechend ausgebildeten Vliesstoff gebildet ist. Alternativ ist möglich, ein separates Klettflauschelement auf der Außenseite des Hauptteils im Vorderbereich vorzusehen, welche als Landezone für die Verschlussmittel der Seitenabschnitte dient.

Obschon die hinteren Seitenabschnitte in der Querrichtung deutlich kürzer sind als bei Gürtelwindeln, erweist es sich als vorteilhaft, wenn die hinteren Seitenabschnitte herstellerseitig um wenigstens zwei in der Längsrichtung verlaufende Seitenabschnitt-Falzachsen auf sich selbst gefaltet sind und die so erhaltene gefaltete Konfiguration herstellerseitig vorzugsweise fixiert ist, etwa durch einzelne Fügestellen, insbesondere Klebe- oder Ultraschallschweißstellen, die gleichwohl zum Entfalten der Seitenabschnitte durch den Benutzer verhältnismäßig leicht manuell lösbar sind. Vorzugsweise in diesem Fall erweist sich ein einziges in der Längsrichtung ungefähr mittig an den Seitenabschnitten positioniertes Verschlussmittel als vorteilhaft, wobei dann die Fügestellen das eingefaltete Verschlussmittel nicht erfassen, sondern in der Längsrichtung außerhalb des Verschlussmittels angeordnet sind. Wenn die gegeneinander anliegenden Teilbereiche um das wenigstens eine nach innen eingeschlagene Verschlussmittel herum oder in der Längsrichtung oberhalb oder unterhalb des eingeschlagenen Verschlussmittels durch die vorgenannten Maßnahmen lösbar fixiert sind, so bildet das eingeschlagene Verschlussmittel einen gut ergreifbaren Anfaßbereich zum Entfalten des jeweiligen Seitenabschnitts.

Weiter erweist sich als vorteilhaft, wenn die hinteren Seitenabschnitte herstellerseitig um wenigstens zwei in der Längsrichtung verlaufende Seitenabschnitt-Falzachsen auf sich selbst gefaltet sind und durch diese Seitenabschnitt-Falzachsen aufeinander gefaltete Teilbereiche der hinteren Seitenabschnitte definiert und begrenzt werden, und dass ein in der Querrichtung außen liegender Teilbereich im wesentlichen undehnbar ausgebildet ist. Hierdurch werden insbesondere die Ergreifbarkeit und die Entfaltbarkeit sowohl des Seitenabschnitts als auch des eingefalteten Verschlussmittels verbessert.

Weiter erweist sich als vorteilhaft, wenn ein an den außenliegenden Teilbereich nach innen anschließender Teilbereich ausgehend von der äußeren in der Längsrichtung verlaufenden Falzachse mit wenigstens 50 % seiner Fläche undehnbar ausgebildet ist. Diese wenigstens 50 % der Fläche werden also ausgehend von der außenliegenden Falzachse bestimmt, indem man hiervon ausgehend eine gedachte parallele Linie zur der Faltachse quasi scannend in der Querrichtung nach innen bewegt bis sie auf einen dehnbaren Bereich stößt. Es wird dann die gescannte Fläche ermittelt und ins Verhältnis zu der Gesamtaufsichtsfläche des Teilbereichs gesetzt. Hierdurch wird erreicht, dass der in der Querrichtung außen liegende Teilbereich und der daran nach innen anschließende Teilbereich über eine sehr große undehnbare Fläche (von wenigstens 50 % der Fläche des letztgenannten Teilbereichs), die demzufolge frei von elastischen oder elastifizierenden Elementen ist, flächenhaft aneinander anliegen.

Die Erstreckung (U) eines jeweiligen undehnbaren Bereichs des an den außenliegenden Teilbereich nach innen anschließenden Teilbereichs beträgt ausgehend von der äußeren in der Längsrichtung verlaufenden Falzachse in Querrichtung bis zum Beginn eines dehnbaren Bereichs vorzugsweise mindestens 15 mm, insbesondere mindestens 20 mm, weiter insbesondere mindestens 25 mm, weiter vorzugsweise mindestens 30 mm, vorzugsweise jedoch höchstens 100 mm, weiter vorzugsweise höchstens 70 mm.

Es wird weiter vorgeschlagen, dass eine jeweilige im ausgefalteten Zustand innenliegende, also dem hinteren seitlichen Längsrand des Hauptteils benachbarte Seitenabschnitt-Falzachse innerhalb des elastischen oder elastifizierten Bereichs des betreffenden Seitenabschnitts verläuft. Hierdurch kann die versteifende und an sich unerwünschte Wirkung einer jeden Faltung bei einem Flachmaterial reduziert werden.

Hingegen erweist es sich als vorteilhaft, wenn eine jeweilige im ausgefalteten Zustand in der Querrichtung weiter außen liegende Seitenabschnitt-Falzachse innerhalb des undehnbaren Bereichs der hinteren Seitenabschnitte verläuft. Dort kann nämlich die versteifende Wirkung der Falzachse durchaus erwünscht sein, da sich hierdurch die Einleitung der Zugkraft über die Verschlussmittel gleichmäßiger auf die Seitenabschnitte verteilt.

Es wird weiter vorgeschlagen, dass die hinteren Seitenabschnitte herstellerseitig um genau zwei in der Längsrichtung verlaufende Seitenabschnitt-Falzachsen auf sich selbst gefaltet sind, so dass genau drei Teilbereiche der Seitenabschnitte gebildet werden, und dass der mittlere Teilbereich ausgehend von der äußeren in der Längsrichtung verlaufenden Falzachse mit wenigstens 50 % seiner Fläche undehnbar ausgebildet ist.

Für die eingefaltete Konfiguration der hinteren Seitenabschnitte gilt vorzugsweise, dass eine Erstreckung
(A) der auf sich selbst gefalteten hinteren Seitenabschnitte in der Querrichtung über den jeweiligen hinteren seitlichen Längsrand hinaus und eine Erstreckung
(B) der auf sich selbst gefalteten hinteren Seitenabschnitte in der Längsrichtung derart bemessen ist, dass das Verhältnis der Erstreckungen (A/B) zueinander 0,5 < A/B <1 beträgt.

Vor der herstellerseitigen Verpackung der Inkontinenzwegwerfwindeln wird vorzugsweise der Hauptteil herstellerseitig mitsamt den auf sich selbst gefalteten hinteren Seitenabschnitten um eine erste und eine zweite jeweils in der Längsrichtung verlaufende Hauptteil-Falzachse nach innen auf sich selbst gefaltet, derart dass die beidseitigen hinteren Seitenabschnitte in Dickenrichtung, also orthogonal zu einer die Längsrichtung und die Querrichtung einschließenden Ebene, in wenigstens teilweiser Überlappung zueinander zu liegen kommen. Weiter vorzugsweise wird vor der herstellerseitigen Verpackung der Inkontinenzwegwerfwindeln der Hauptteil herstellerseitig mitsamt den auf sich selbst gefalteten hinteren Seitenabschnitten und vorzugsweise im Anschluss an die zuvor beschriebene Faltung um in der Längsrichtung verlaufende Hauptteil-Falzachsen zusätzlich um eine oder zwei in der Querrichtung verlaufende Hauptteil-Falzachsen nach innen auf sich selbst gefaltet.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform der erfindungsgemäßen Inkontinenzwegwerfwindel. In der Zeichnung zeigt:
Figur 1 eine Draufsicht auf eine erfindungsgemäße Inkontinenzwegwerfwindel im eben ausgebreiteten, jedoch nicht gedehnten Zustand;
Figur 2 eine Schnittansicht der Windel nach Figur eins mit Schnittebene II-II;
Figur 3 eine schematische Darstellung der Windel im angelegten Zustand;
Figuren 4a, b, c jeweils eine vergrößerte und teilweise Darstellung der Windel nach Figur 1 im Bereich eines hinteren Seitenabschnitts in eben ausgebreitetem, jedoch nicht gedehnten Zustand mit Bemaßungen bzw. mit Falzachsen;
Figur 5 eine vergrößerte Darstellung der Windel nach Figur 1 im Bereich eines hinteren Seitenabschnitts in auf sich selbst gefalteter Konfiguration;
Figur 6 eine Schnittansicht mit Schnittebene VI-VI Figur 5;
Figuren 7 und 8 eine Darstellung der Windel entsprechend Figur 4 mit Klemmen einer Vorrichtung zur Bestimmung der Dehnbarkeit.

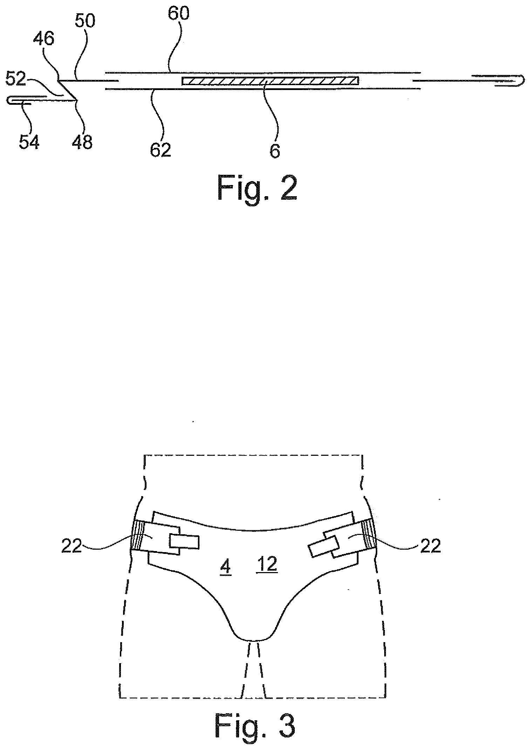

Die Figuren zeigen eine erfindungsgemäße insgesamt mit dem Bezugszeichen 2 bezeichnete Inkontinenzwegwerfwindel in der sogenannten T-Form. Die Windel 2 umfasst einen insgesamt mit dem Bezugszeichen 4 bezeichneten Hauptteil mit einem Körperflüssigkeiten absorbierenden Saugkörper 6. Der Saugkörper 6 umfasst vorzugsweise Zellulosefasern und superabsorbierende Polymerpartikel (SAP). Bei der Windel 2 ist eine Längsrichtung 8 und einer Querrichtung 10 unterscheidbar, wobei letztere im angelegten Zustand der Windel der Hüftumfangsrichtung des Benutzers entspricht. Der Hauptteil 4 umfasst einen Vorderbereich 12 mit vorderen seitlichen Längsrändern 14, einen Rückenbereich 16 mit hinteren seitlichen Längsrändern 18 und einen dazwischen angeordneten Schrittbereich 20. Bei der T-förmigen Windel 2 sind nur im Rückenbereich 16 des Hauptteils 4 in Querrichtung 10 seitlich über die hinteren seitlichen Längsränder 18 hinaus erstreckte hintere Seitenabschnitte 22 vorgesehen, die im Bereich der hinteren seitlichen Längsränder 18 in einem Überlappungsbereich 24 unlösbar an den Rückenbereich 16 des Hauptteils 4 angefügt sind. Die hinteren Seitenabschnitte 22 haben im Bereich ihres in Querrichtung 10 freien Endes 26 jeweils wenigstens ein Verschlussmittel 28. Das Verschlussmittel 28 ist in Form einer vorzugsweise rechteckigen Lasche ausgebildet und auf sich selbst eingefaltet. In der Gebrauchssituation lässt sich das Verschlussmittel öffnen, das heißt wieder auffalten, um die Inkontinenzwegwerfwindel 2 an einen Benutzer anzulegen, wobei die Seitenabschnitte 22 in Überlappung mit dem Vorderbereich 12 des Hauptteils 4 gebracht werden und die Verschlussmittel lösbar haftend auf der Außenseite des Vorderteils des Hauptteils festgelegt werden (schematisch dargestellt in Figur 3).

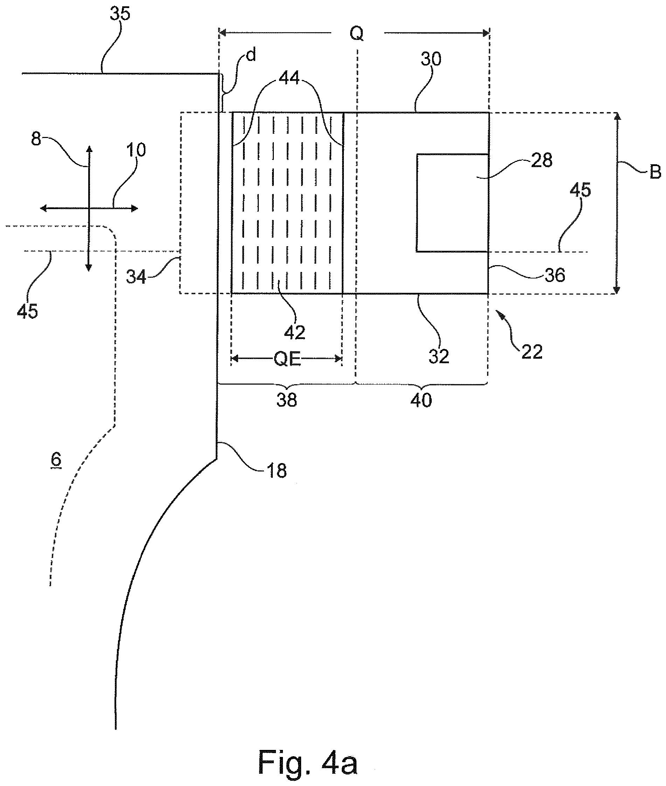

Die Seitenabschnitte 22 sind - wie am besten aus Figur 4a ersichtlich ist - vorzugsweise rechteckförmig ausgebildet, wobei sie durch in der Querrichtung 10 verlaufende Ränder 30, 32 und durch in der Längsrichtung 8 verlaufende Ränder 34, 36 begrenzt werden. In dem in Figur 4a dargestellten eben ausgefalteten oder ausgebreiteten jedoch nicht gedehnten Zustand haben die hinteren Seitenabschnitte 22 eine Erstreckung Q von 200 mm in der Querrichtung 10 über den hinteren seitlichen Längsrand 18 hinaus. Diese Erstreckung Q der Seitenabschnitte 22 in Querrichtung 10 außerhalb des Hauptteils 4 umfasst eine an den hinteren seitlichen Längsrand 18 anschließende proximale Hälfte 38 und eine frei endende distale Hälfte 40 der Seitenabschnitte 22. Die Seitenabschnitte 22 haben in der Längsrichtung 8 einen Abstand d von vorzugsweise 5-50 mm von einem hinteren Querrand 35 der Windel. Die Erstreckung (B) der Seitenabschnitte in der Längsrichtung beträgt im dargestellten Fall 140 mm.

Die Seitenabschnitte 22 sind in Querrichtung 10 außerhalb des Hauptteils 4 elastisch dehnbar ausgebildet. Sie umfassen hierfür einen elastischen oder elastifizierten Bereich 42. Dieser elastische oder elastifizierte Bereich 42 der hinteren Seitenabschnitte 22 ist vollständig innerhalb der proximalen Hälfte 38 der hinteren Seitenabschnitte 22 angeordnet. Er weist im beispielhaft dargestellten Fall in der Querrichtung 10 einen geringen Abstand von wenigen Millimetern von dem hinteren seitlichen Längsrand 18 auf. Der elastische oder elastifizierte Bereich 42 ist rechteckförmig ausgebildet und seine Erstreckung (QE) in der Querrichtung ist durch in der Längsrichtung 8 erstreckte Ränder 44 begrenzt, wobei die Ränder 44 sich über die gesamte Längserstreckung der hinteren Seitenabschnitte 22 erstrecken.

In der distalen Hälfte 40 der Erstreckung Q der hinteren Seitenabschnitte 22 sind die hinteren Seitenabschnitte in der Querrichtung 10 undehnbar ausgebildet.

Typischerweise kann der jeweilige elastische oder elastifizierte Bereich 42 der hinteren Seitenabschnitte 22 durch Zwischenordnung eines elastisch dehnbaren oder eines elastifizierten Materials in die Seitenabschnitte 22 erreicht werden. Die Seitenabschnitte 22 sind dann durch in der Querrichtung 10 hintereinander angeordnete und miteinander gefügte Materialabschnitte verschiedener Dehnbarkeit ausgebildet. Alternativ hierzu können elastifizierende Mittel im vorgespannten Zustand mit Flachmaterialien der hinteren Seitenabschnitte 22 verbunden werden. Weiter alternativ ist es möglich, dass an sich undehnbare Flachmateriallaminate, wie beispielsweise Laminate umfassend undehnbare Vliesstoffe und daran flächenhaft angefügte elastische Folien der hinteren Seitenabschnitte 22 durch im Stand der Technik bekannte Maßnahmen, etwa durch das sogenannte "RingRolling", bereichsweise "aktiviert", das heißt elastisch dehnbar gemacht werden.

Die hinteren Seitenabschnitte 22 sind weiter derart am Rückenbereich 16 des Hauptteils 4 angeordnet, dass eine in der Querrichtung 10 erstreckte und das jeweilige Verschlussmittel 28 auf der schrittzugewandten Seite tangierende gerade Linie 45 den Saugkörper 6 schneidet.

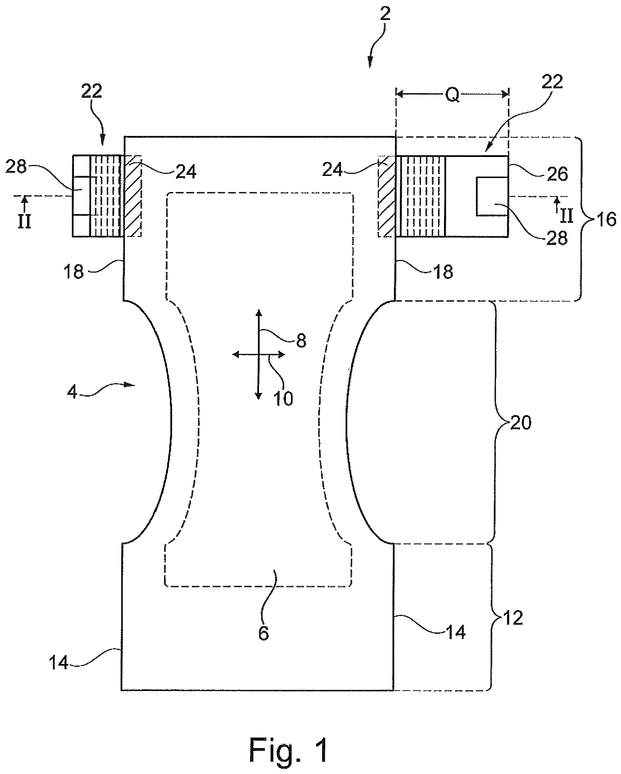

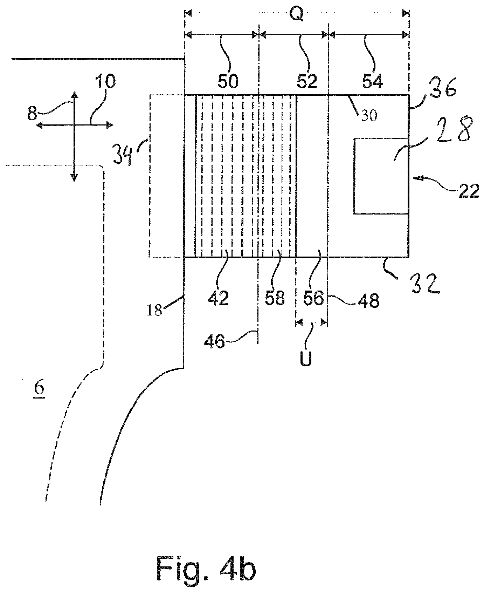

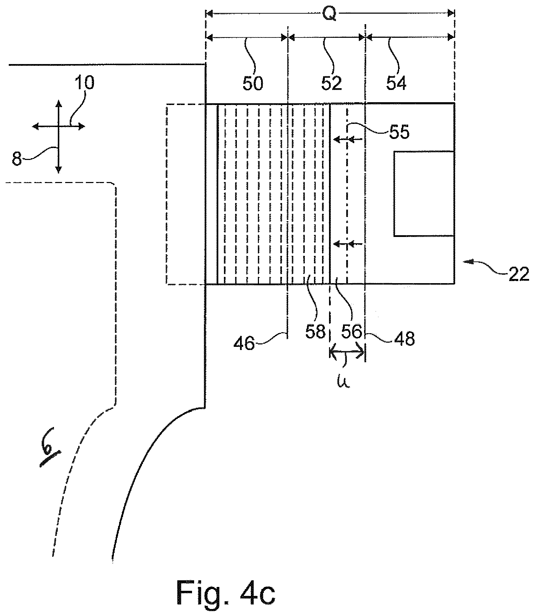

Wie in Figuren 1, 2 (jeweils links) und Figuren 4b, 5 und 6 dargestellt sind die hinteren Seitenabschnitte 22 herstellerseitig um wenigstens zwei in der Längsrichtung 8 verlaufende Seitenabschnitt-Falzachsen 46, 48 auf sich selbst gefaltet. Die Seitenabschnitt-Falzachsen 46, 48 definieren und begrenzen dabei aufeinander gefaltete Teilbereiche 50, 52, 54 der hinteren Seitenabschnitte 22 (Figur 4b). Man erkennt, dass die dem hinteren seiltichen Längsrand 18 benachbarte, also innenliegende Seitenabschnitt-Falzachse 46 innerhalb des elastischen oder elastifizierten Bereichs 42 verläuft, während die in der Querrichtung 10 weiter außen liegende Seitenabschnitt-Falzachse 48 außerhalb des elastischen oder elastifizierten Bereichs 42, also innerhalb eines undehnbaren Bereichs der Seitenabschnitte 22 verläuft. Der Teilbereich 52, welcher sich an den in der Querrichtung 10 außenliegenden Teilbereich 54 nach innen anschließt, ist ausgehend von der äußeren in der Längsrichtung 8 verlaufenden Falzachse 48 mit wenigstens 50 % seiner Fläche undehnbar ausgebildet. Zur Bestimmung dieser undehnbaren Fläche wird von der äußeren Falzachse 48 ausgehend und parallel hierzu eine gedachte Linie 55 quasi scannend in der Querrichtung 10 in Richtung auf den Hauptteil 4 nach innen bewegt (dies ist in Figur 4c durch Pfeile veranschaulicht) bis sie auf einen dehnbaren Bereich 58 stößt. Die so gescannte Fläche beträgt wenigstens 50 % der gesamten Aufsichtsfläche des Teilbereichs 52 im nicht gedehnten Zustand. Der Teilbereich 52 weist hier einen rechteckförmigen in der Querrichtung 10 äußeren undehnbaren Bereich 56 einer Quererstreckung U und einen rechteckförmigen in der Querrichtung 10 inneren elastisch dehnbaren Bereich 58 auf, die durch eine in der Längsrichtung 8 verlaufende gedachte Linie voneinander abgegrenzt sind. Dabei umfasst der rechteckförmige äußere undehnbare Bereich 56 - wie ausgeführt - wenigstens die Hälfte der gesamten Aufsichtsfläche des Teilbereichs 52 im nicht gedehnten Zustand. Es ist auch denkbar, dass der sich an den außen liegenden Teilbereich 54 nach innen anschließende Teilbereich 52 über seine gesamte Erstreckung undehnbar ausgebildet ist.

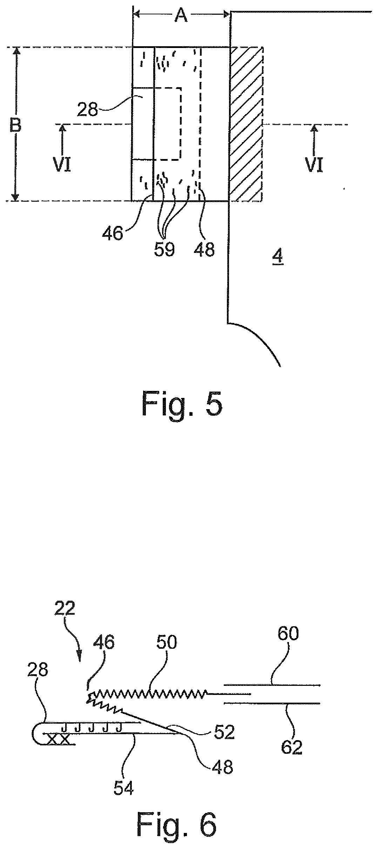

Die Figuren 5 und 6 zeigen schematisch die auf sich selbst gefaltete Konfiguration der hinteren Seitenabschnitte 22. Die gegeneinander gefalteten Teilbereiche 50, 52, 54 sind an in Figur 5 dargestellten Fügestellen 59 lösbar aneinander fixiert. Diese Fügestellen 59 sind durch eingangs beschriebene Maßnahmen ausgebildet. Es erweist sich als vorteilhaft, dass im Bereich der Überlappung der Teilbereiche 52, 54 zu einem wesentlichen Anteil undehnbare Bereiche miteinander lösbar gefügt sind. Man erkennt in Figur 6 auch in schematischer Darstellung die Anfügung des Verschlussmittels 28 beispielhaft an die körperabgewandte Außenseite des Seitenabschnitts 22 und die Umfaltung auf die körperzugewandte Seite des Seitenabschnitts 22. Ferner erkennt man die schematisch dargestellte Anfügung der hinteren Seitenabschnitte 22 beispielhaft zwischen zwei Flachmaterialien, beispielsweise eine flüssigkeitsdurchlässige Deckschicht 60 (Topsheet) und eine flüssigkeitsundurchlässige Rückschicht 62 (Backsheet) des Windelhauptteils 4.

Die Abmessung A der gefalteten Konfiguration der hinteren Seitenabschnitte 22 in der Querrichtung 10 außerhalb des Hauptteils 4 und die Abmessung B in der Längsrichtung 8 sind in Figur 5 dargestellt. Das Verhältnis A/B beträgt vorzugsweise 0,5 < A/B < 1. Das entsprechende Verhältnis Q/B der Seitenabschnitte 22 im eben ausgebreiteten jedoch nicht gedehnten Zustand beträgt vorzugsweise 1,0 < Q/B < 2,0 und ist in Figur 4a dargestellt. Das Verhältnis von QE, also der Erstreckung des elastischen oder elastifizierten Bereichs 42 in der Querrichtung, zu Q beträgt vorzugsweise 0,20 < QE/Q < 0,50, insbesondere 0,30 < QE/Q < 0,45.

Sofern es sich im Einzelfall als nicht offensichtlich erweisen sollte, ob ein Seitenabschnittsbereich elastisch dehnbar oder undehnbar ist, wird zur quantitativen Abgrenzung der Begriffe undehnbar, dehnbar bzw. elastisch dehnbar das nachfolgende Testverfahren angegeben:
Die Dehnbarkeit kann hierbei direkt am Seitenabschnitt 22 der Windel bestimmt werden. Hierfür wird ein betreffender Bereich eines Seitenabschnitts 22 zwischen zwei Klemmbacken 70, 72 von definierter, gleicher Klemmbackenbreite b von 50 mm eingespannt, wobei der Klemmbackenabstand a 30 mm beträgt. Dabei erstreckt sich die Klemmbackenbreite b in der Längsrichtung 8 und der Klemmbackenabstand a in der Querrichtung 10, wobei sich der Seitenabschnitt 22 in eben ausgebreitetem jedoch nicht gedehnten Zustand befindet. Sofern der zu erfassende Bereich in der Querrichtung 10 eine Erstreckung von weniger als 30 mm haben sollte, wird der Klemmbackenabstand a entsprechend geringer gewählt. Die Prüfung erfolgt ausgehend von einer Vorkraft von 0,2 N zwischen den Klemmbacken 70, 72. Die Klemmbacken 70, 72 werden hiervon ausgehend mit einer konstanten Geschwindigkeit von 100 mm/min bis zum Erreichen einer Kraft von 15 N in der Querrichtung 10 voneinander weg bewegt und im wesentlichen unmittelbar nach Erreichen der Kraft von 15 N wieder aufeinander zu bewegt, und zwar wiederum mit einer konstanten Geschwindigkeit von 100 mm/min bis zum Erreichen des Werts der Vorkraft von 0,2 N. Es wird zum einen der initiale Klemmbackenabstand L0 in mm bei Erreichen der Vorkraft von 0,2 N notiert. Des Weiteren wird der Klemmbackenabstand L1 in mm bei Erreichen der Kraft von 15 N notiert (vorzugsweise erfolgt dies jeweils automatisiert über die Auswerteeinheit der Prüfvorrichtung). Ferner wird am Ende der Prüfung der verbleibende Klemmbackenabstand L2 in mm nach Zurücknahme der Kraft auf den Wert der Vorkraft von 0,2 N notiert.

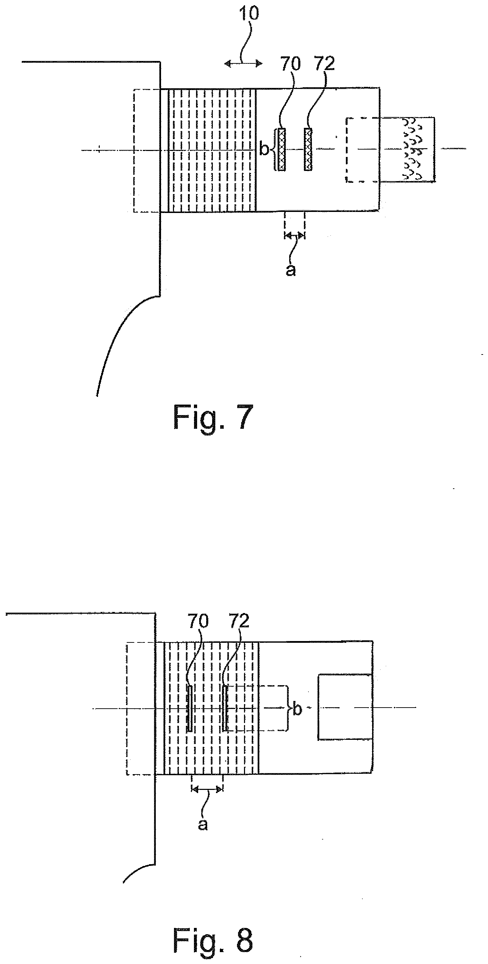

Im Falle der Prüfung der Dehnbarkeit im Bereich der distalen Hälfte 42 der hinteren Seitenabschnitte 22 werden die Klemmbacken 70, 72 vorzugsweise derart positioniert, dass sie außerhalb von Verschlussmitteln angeordnet sind. Hierfür kann wie in Figur 7 dargestellt das Verschlussmittel nach außen ausgefaltet werden. Die Prüfung der von dem Verschlussmittel überfangenen Fläche ist nicht erforderlich, da die Seitenabschnitte dort aufgrund der Verschlussmittel in der Regel ohnehin undehnbar sind.

Im Falle der Prüfung der elastischen Dehnbarkeit innerhalb der proximalen Hälfte 38 der Seitenabschnitte 22 werden die Klemmbacken 70, 72 vorzugsweise in der Längsrichtung 8 mittig bezüglich der Längserstreckung der Seitenabschnitte 22 angeordnet, so wie dies in Figur 8 dargestellt ist. Es wird dann die vorstehende Prüfung ausgeführt. Im Anschluss daran werden die Klemmbacken 70, 72 in der Längsrichtung 8 versetzt (in der Figur 8 nach oben bzw. unten versetzt), so dass in der Längsrichtung 8 angrenzende Bereiche des Seitenabschnitts erfasst werden können und der Seitenabschnitt somit über seine gesamte Erstreckung in der Längsrichtung 8 getestet werden kann.

Für die Auswertung wird unter dem Begriff der Dehnung das Verhältnis zwischen einer Zunahme des Klemmbackenabstands bei Erreichen von 15 N und dem Klemmbackenabstand bei der Vorkraft von 0,2 N verstanden, also gedehnter Klemmbackenabstand bei 15 N L1 in mm minus L0 in mm geteilt durch L0 in mm, also Dehnung [%] = (L1 - L0) / L0.

Für die Ermittlung der bleibenden Dehnung (permanent set) wird das Verhältnis der Zunahme des Klemmbackenabstands nach Zurücknahme der Kraft auf den Wert der Vorkraft von 0,2 N gegenüber dem initialen Klemmbackenabstand (L2 - L0) und des initialen Klemmbackenabstands L0 verstanden, also bleibende Dehnung [%] = (L2 - L0) / L0.

Im Rahmen der vorliegenden Erfindung wird die distale Hälfte 40 oder ein beliebiger Bereich eines jeweiligen hinteren Seitenabschnitts 22 als undehnbar angesehen, wenn bei Ausführung des vorstehenden Tests jeder in der Längsrichtung 8 50 mm und in der Querrichtung 10 30 mm erstreckte Bereich der distalen Hälfte 40 bei kurzzeitiger Einwirkung einer Kraft von 15 N eine Dehnung von weniger als 50 % zulässt.

Im Rahmen der vorliegenden Erfindung wird die proximale Hälfte 38 oder ein Bereich 42 der proximalen Hälfte 38 oder ein beliebiger Bereich eines jeweiligen hinteren Seitenabschnitts 22 als elastisch dehnbar angesehen, wenn bei Ausführung des vorstehenden Tests der von den Klemmbacken erfasste Bereich bei 15 N eine Dehnung von wenigstens 60 % zulässt und nach Zurücknahme der Kraft auf den Wert der Vorkraft von 0,2 N eine bleibende Dehnung (permanent set) von höchstens 15 % verbleibt, und zwar auch bei Ausführung des Tests mit in der Längsrichtung 8 versetzten Klemmbacken. Es wird also der Seitenabschnitt 22 über seine gesamte Erstreckung in der Längsrichtung 8 getestet.

Im Falle, dass das zwischen den Klemmbacken eingespannte Material eines Seitenabschnitts bei der Ausführung des vorstehenden Tests bricht, bevor die maximale Kraft von 15 N erreicht ist und die Bruchkraft somit weniger als 15N/50mm beträgt, wird das Material als nicht zur Ausführung der Erfindung geeignet angesehen.

## Patentansprüche

1. Inkontinenzwegwerfwindel (2), mit einem einen Saugkörper (6) aufweisenden Hauptteil (4) mit einer Längsrichtung (8) und einer Querrichtung (10), umfassend einen Vorderbereich (12) mit vorderen seitlichen Längsrändern (14), einen Rückenbereich (16) mit hinteren seitlichen Längsrändern (18) und einen dazwischen angeordneten, zwischen den Beinen eines Benutzers zu liegen kommenden Schrittbereich (20), und mit beidseits an den Rückenbereich (16) angefügten hinteren Seitenabschnitten (22), welche sich in der Querrichtung (10) der Inkontinenzwegwerfwindel (2) über die seitlichen hinteren Längsränder (18) des Hauptteils (4) hinaus erstrecken und im Bereich ihres in der Querrichtung (10) freien Endes (26) jeweils wenigstens ein Verschlussmittel (28) tragen, wohingegen an den Vorderbereich (14) keine Seitenabschnitte (22) angefügt sind, sondern die vorderen seitlichen Längsränder (14) des Hauptteils (4) einen frei endenden Längsrand der Windel bilden, wobei die hinteren Seitenabschnitte (22) zum Anlegen und Schließen der Inkontinenzwegwerfwindel (2) an einen Benutzer jeweils entlang einer Umfangsrichtung um den Körper des Benutzers herumlegbar und in überlappende Anordnung mit einer Außenseite des Vorderbereichs (12) bringbar sind, an der sie dann über das jeweilige Verschlussmittel (28) jeweils lösbar anhaftbar sind, wobei die hinteren Seitenabschnitte (22) in eben ausgebreitetem jedoch nicht gedehnten Zustand eine Erstreckung (Q) in der Querrichtung (10) über den jeweiligen hinteren seitlichen Längsrand (18) hinaus aufweisen und innerhalb dieser Erstreckung (Q) in der Querrichtung (10) elastisch dehnbar sind und hierfür einen in der Querrichtung (10) und in der Längsrichtung (8) erstreckten elastischen oder elastifizierten Bereich (42) aufweisen, wobei diese Erstreckung (Q) der hinteren Seitenabschnitte (22) in der Querrichtung (10) eine an den hinteren seitlichen Längsrand (18) anschließende proximale Hälfte (38) und eine frei endende distale Hälfte (40) umfasst, **dadurch gekennzeichnet, dass** der jeweilige elastische oder elastifizierte Bereich (42) der hinteren Seitenabschnitte (22) vollständig innerhalb der proximalen Hälfte (38) angeordnet ist und an den zugeordneten hinteren seitlichen Längsrand (18) heranreicht oder einen Abstand von dem hinteren seitlichen Längsrand (18) in der Querrichtung von höchstens 30 mm aufweist, und dass die jeweiligen hinteren Seitenabschnitte (22) in der gesamten distalen Hälfte (40) im wesentlichen undehnbar ausgebildet sind.

2. Inkontinenzwegwerfwindel nach Anspruch 1, **dadurch gekennzeichnet, dass** der jeweilige elastische oder elastifizierte Bereich der hinteren Seitenabschnitte (22) an den zugeordneten hinteren seitlichen Längsrand (18) heranreicht, jedoch ohne den hinteren seitlichen Längsrand zu überlappen.

3. Inkontinenzwegwerfwindel nach Anspruch 1 oder 2, **dadurch gekennzeichnet, dass** die beiden hinteren Seitenabschnitte (22) mit einem undehnbaren den Hauptteil (4) überlappenden Bereich (24) an den Hauptteil (4) unlösbar angefügt sind.

4. Inkontinenzwegwerfwindel nach Anspruch 1, 2 oder 3, **dadurch gekennzeichnet, dass** der jeweilige elastische oder elastifizierte Bereich (42) in der Querrichtung (10) um wenigstens 70 %, insbesondere um wenigstens 80 %, weiter insbesondere um wenigstens 90 % dehnbar ist.

5. Inkontinenzwegwerfwindel nach einem oder mehreren der vorstehenden Ansprüche, **dadurch gekennzeichnet, dass** die Erstreckung (Q) der hinteren Seitenabschnitte (22) in der Querrichtung (10) über den jeweiligen hinteren seitlichen Längsrand (18) hinaus und eine maximale Erstreckung (B) der hinteren Seitenabschnitte (22) in der Längsrichtung (8) derart bemessen ist, dass das Verhältnis der Erstreckungen (Q/B) zueinander 1,0 < Q/B < 2,0 beträgt.

6. Inkontinenzwegwerfwindel nach einem oder mehreren der vorstehenden Ansprüche, **dadurch gekennzeichnet, dass** die beiden hinteren Seitenabschnitte (22) in der Längsrichtung (8) einen Abstand (d) zu einem hinteren Querrand (35) des Hauptteils (4) von wenigstens 1 mm, insbesondere von wenigstens 5 mm, insbesondere von wenigstens 10 mm, insbesondere von wenigstens 15 mm, insbesondere von höchstens 50 mm aufweisen.

7. Inkontinenzwegwerfwindel nach einem oder mehreren der vorstehenden Ansprüche, **dadurch gekennzeichnet, dass** eine in der Querrichtung erstreckte und das jeweilige Verschlussmittel auf der schrittzugewandten Seite tangierende gerade Linie (45) den Saugkörper (6) schneidet.

8. Inkontinenzwegwerfwindel nach einem oder mehreren der vorstehenden Ansprüche, **dadurch gekennzeichnet, dass** jeder hintere Seitenabschnitt (22) genau ein Verschlussmittel (28) aufweist.

9. Inkontinenzwegwerfwindel nach einem oder mehreren der vorstehenden Ansprüche, **dadurch gekennzeichnet, dass** die hinteren Seitenabschnitte (22) herstellerseitig um wenigstens zwei in der Längsrichtung (8) verlaufende Seitenabschnitt-Falzachsen (46, 48) auf sich selbst gefaltet sind und durch diese Seitenabschnitt-Falzachsen (46, 48) aufeinander gefaltete Teilbereiche (50, 52, 54) der hinteren Seitenabschnitte (22) definiert und begrenzt werden, und dass ein in der Querrichtung (10) außenliegender Teilbereich (54) im wesentlichen undehnbar ausgebildet ist.

10. Inkontinenzwegwerfwindel nach Anspruch 9, **dadurch gekennzeichnet, dass** ein an den außenliegenden Teilbereich (54) nach innen anschließender Teilbereich (52) ausgehend von der äußeren in der Längsrichtung verlaufenden Falzachse (48) mit wenigstens 50 % seiner Fläche undehnbar ausgebildet ist.

11. Inkontinenzwegwerfwindel nach Anspruch 9 oder 10, **dadurch gekennzeichnet, dass** eine jeweilige im ausgefalteten Zustand innenliegende, also dem hinteren seitlichen Längsrand (18) des Hauptteils (4) benachbarte Seitenabschnitt-Falzachse (46) innerhalb des elastischen oder elastifizierten Bereichs (42) des betreffenden Seitenabschnitts (22) verläuft.

12. Inkontinenzwegwerfwindel nach Anspruch 9, 10 oder 11, **dadurch gekennzeichnet, dass** eine jeweilige im ausgefalteten Zustand in der Querrichtung (10) weiter außen liegende Seitenabschnitt-Falzachse (48) innerhalb eines undehnbaren Bereichs der hinteren Seitenabschnitte (22) verläuft.

13. Inkontinenzwegwerfwindel nach einem oder mehreren der vorstehenden Ansprüche, **dadurch gekennzeichnet, dass** die hinteren Seitenabschnitte (22) herstellerseitig um genau zwei in der Längsrichtung (8) verlaufende Seitenabschnitt-Falzachsen (46, 48) auf sich selbst gefaltet sind, so dass genau drei Teilbereiche (50, 52, 54) der Seitenabschnitte (22) gebildet werden, und dass der mittlere Teilbereich (52) ausgehend von der äußeren in der Längsrichtung (8) verlaufenden Falzachse (48) mit wenigstens 50 % seiner Fläche undehnbar ausgebildet ist.

14. Inkontinenzwegwerfwindel nach einem oder mehreren der Ansprüche 9-13, **dadurch gekennzeichnet, dass** eine Erstreckung (A) der auf sich selbst gefalteten hinteren Seitenabschnitte (22) in der Querrichtung (10) über den jeweiligen hinteren seitlichen Längsrand (18) hinaus und eine Erstreckung (B) der auf sich selbst gefalteten hinteren Seitenabschnitte (22) in der Längsrichtung (8) derart bemessen ist, dass das Verhältnis der Erstreckungen (A/B) zueinander 0,5 < A/B < 1 beträgt.

15. Inkontinenzwegwerfwindel nach einem oder mehreren der Ansprüche 9-14, **dadurch gekennzeichnet, dass** der Hauptteil (4) herstellerseitig mitsamt den auf sich selbst gefalteten hinteren Seitenabschnitten (22) um eine erste und eine zweite jeweils in der Längsrichtung (8) verlaufende Hauptteil-Falzachse nach innen auf sich selbst gefaltet ist, derart dass die beidseitigen hinteren Seitenabschnitte (22) in Dickenrichtung in wenigstens teilweiser Überlappung zueinander zu liegen kommen.

## Claims

1. A disposable incontinence diaper (2), having a main part (4) having an absorbent element (6) and a longitudinal direction (8) and a transverse direction (10), comprising a front region (12) having forward lateral longitudinal peripheries (14), a back region (16) having rearward lateral longitudinal peripheries (18), and disposed therebetween a crotch region (20) that comes to lie between the legs of a user, and rearward side portions (22) which are joined to both sides of the back region (16) and which in the transverse direction (10) of the disposable incontinence diaper (2) extend beyond the lateral rearward longitudinal peripheries (18) of the main part (4) and in the region of that end (26) of said rearward side portions (22) that is free in the transverse direction (10) have in each case at least one closure means (28), whereas no side portions (22) are joined to the front region (14) but the forward lateral longitudinal peripheries (14) of the main part (4) form a free-ending longitudinal periphery of the diaper, wherein the rearward side portions (22) for placing and closing the disposable incontinence diaper (2) on a user are in each case capable of being placed around the body of the user in a circumferential direction and of being moved to an arrangement that overlaps with an external side of the front region (12), said rearward side portions (22) then being capable of being in each case releasably adhered to said external side of the front region (12) by way of the respective closure means (28), wherein the rearward side portions (22) in a planar spread-out but not elongated state have an extent (Q) in the transverse direction (10) beyond the respective rearward lateral longitudinal periphery (18) and within this extent (Q) are elastically elongatable in the transverse direction (10) and to this end have an elastic or elasticized region (42) that extends in the transverse direction (10) and in the longitudinal direction (8), wherein this extent (Q) in the transverse direction (10) of the rearward side portions (22) comprises a proximal half (38) that adjoins the rearward lateral longitudinal periphery (18) and a free-ending distal half (40), **characterized in that** the respective elastic or elasticized region (42) of the rearward side portions (22) is disposed completely within the proximal half (38) and reaches up to the assigned rearward lateral longitudinal periphery (18), or has a spacing in the transverse direction from the rearward lateral longitudinal periphery (18) of at most 30 mm, and **in that** the respective rearward side portions (22) in the entire distal half (40) are configured so as to be substantially non-elongatable.

2. The disposable incontinence diaper as claimed in claim 1, **characterized in that** the respective elastic or elasticized region of the rearward side portions (22) reaches up to the assigned rearward lateral longitudinal periphery (18), without however overlapping the rearward lateral longitudinal periphery.

3. The disposable incontinence diaper as claimed in claim 1 or 2, **characterized in that** the two rearward side portions (22), conjointly with a non-elongatable region (24) that overlaps the main part (4), are non-releasably joined to the main part (4).

4. The disposable incontinence diaper as claimed in claim 1, 2 or 3, **characterized in that** the respective elastic or elasticized region (42) in the transverse direction (10) is elongatable by at least 70%, in particular by at least 80%, furthermore particularly by at least 90%.

5. The disposable incontinence diaper as claimed in one or a plurality of the preceding claims, **characterized in that** the extent (Q) in the transverse direction (10) of the rearward side portions (22) beyond the respective rearward lateral longitudinal periphery (18), and a maximum extent (B) in the longitudinal direction (8) of the rearward side portions (22), are dimensioned such that the mutual ratio of the extents (Q/B) is 1.0 < Q/B < 2.0.

6. The disposable incontinence diaper as claimed in one or a plurality of the preceding claims, **characterized in that** the two rearward side portions (22) in the longitudinal direction (8) have a spacing (d) from a rearward transverse periphery (35) of the main part (4) of at least 1 mm, in particular of at least 5 mm, in particular of at least 10 mm, in particular of at least 15 mm, in particular of at most 50 mm.

7. The disposable incontinence diaper as claimed in one or a plurality of the preceding claims, **characterized in that** a straight line (45) that extends in the transverse direction and is tangent to the respective closure means on the crotch-facing side intersects the absorbent element (6).

8. The disposable incontinence diaper as claimed in one or a plurality of the preceding claims, **characterized in that** each rearward side portion (22) has exactly one closure means (28).

9. The disposable incontinence diaper as claimed in one or a plurality of the preceding claims, **characterized in that** the rearward side portions (22) ex works are folded onto themselves about at least two side portion folding axes (46, 48) that run in the longitudinal direction (8), part-regions (50, 52, 54) of the rearward side portions (22) that are folded onto one another being defined and delimited by said side portion folding axes (46, 48), and **in that** a part-region (54) that is outboard in the transverse region (10) is configured so as to be substantially non-elongatable.

10. The disposable incontinence diaper as claimed in claim 9, **characterized in that** a part-region (52) that inwardly adjoins the outboard part-region (54), proceeding from the outer folding axis (48) that runs in the longitudinal direction, by way of at least 50% of the area of said part-region (52) is configured so as to be non-elongatable.

11. The disposable incontinence diaper as claimed in claim 9 or 10, **characterized in that** a respective side portion folding axis (46) that in the unfolded state is inboard, thus adjacent to the rearward lateral longitudinal periphery (18) of the main part (4), runs within the elastic or elasticized region (42) of the respective side portion (22).

12. The disposable incontinence diaper as claimed in claim 9, 10, or 11, **characterized in that** a respective side portion folding axis (48) that in the unfolded state is further outboard in the transverse direction (10) runs within a non-elongatable region of the rearward side portions (22).

13. The disposable incontinence diaper as claimed in one or a plurality of the preceding claims, **characterized in that** the rearward side portions (22) ex works are folded onto themselves about exactly two side portion folding axes (46, 48) that run in the longitudinal direction (8) such that exactly three part-regions (50, 52, 54) of the side portions (22) are formed, and **in that** the central part-region (52), proceeding from the outer folding axis (48) that runs in the longitudinal direction (8), by way of at least 50% of the area thereof is configured so as to be non-elongatable.

14. The disposable incontinence diaper as claimed in one or a plurality of claims 9-13, **characterized in that** an extent (A) in the transverse direction (10) of the rearward side portions (22) that are folded onto themselves beyond the respective rearward lateral longitudinal periphery (18), and an extent(B) in the longitudinal direction (8) of the rearward side portions (22) that are folded onto themselves, are dimensioned such that the mutual ratio of the extents (A/B) is 0.5 < A/B < 1.

15. The disposable incontinence diaper as claimed in one or a plurality of claims 9-14, **characterized in that** the main part (4), together with the rearward side portions (22) that are folded inward onto themselves, is ex works folded onto itself about a first and a second main part folding axis that in each case run in the longitudinal direction (8) in such a manner that the rearward side portions (22) on both sides come to lie so as to at least partially overlap one another in the direction of thickness.

## Revendications

1. Couche jetable pour incontinence (2) comprenant une partie principale (4) présentant un corps absorbant (6) et ayant une direction longitudinale (8) et une direction transversale (10), comprenant une zone avant (12) ayant des bords longitudinaux latéraux avant (14), une zone dorsale (16) ayant des bords longitudinaux latéraux arrière (18) et une zone d'entrejambe (20) agencée entre celles-ci et venant se positionner entre les jambes d'un utilisateur, et avec des portions latérales arrière (22) raccordées de part et d'autre à la zone dorsale (16), qui s'étendent dans la direction transversale (10) de la couche jetable pour incontinence (2) au-delà des bords longitudinaux latéraux arrière (18) de la partie principale (4) et qui portent chacune au moins un moyen de fermeture (28) au niveau de leur extrémité (26) libre dans la direction transversale (10), tandis qu'aucune portion latérale (22) n'est raccordée à la zone avant (14), mais que les bords longitudinaux latéraux avant (14) de la partie principale (4) forment un bord longitudinal de la couche lequel se termine librement, dans laquelle les portions latérales arrière (22) destinées à appliquer et à fermer la couche jetable pour incontinence (2) à ou bien sur un utilisateur peuvent être appliquées chacune suivant une direction circonférentielle autour du corps de l'utilisateur et peuvent être mises en disposition à chevauchement avec une face extérieure de la zone avant (12), à laquelle elles peuvent être attachées ensuite respectivement de manière amovible par le moyen de fermeture (28) respectif, dans laquelle les portions latérales arrière (22), lorsqu'elles se trouvent dans un état déployé de manière plane, mais non pas étiré, présentent une extension (Q) dans la direction transversale (10) au-delà du bord longitudinal latéral arrière (18) respectif, et sont extensibles de manière élastique dans la direction transversale (10) à l'intérieur de cette extension (Q) et présentent, à cette fin, une zone (42) élastique ou élastifiée s'étendant dans la direction transversale (10) et dans la direction longitudinale (8), cette extension (Q) des portions latérales arrière (22) dans la direction transversale (10) comprenant une moitié proximale (38) contiguë au bord longitudinal latéral arrière (18) ainsi qu'une moitié distale (40) se terminant librement, **caractérisée par le fait que** la zone (42) élastique ou élastifiée respective des portions latérales arrière (22) est disposée complètement à l'intérieur de la moitié proximale (38) et s'étend jusqu'au bord longitudinal latéral arrière (18) associé ou présente une distance de 30 mm tout au plus par rapport au bord longitudinal latéral arrière (18) dans la direction transversale, et que les portions latérales arrière (22) respectives sont conçues de manière à être pour l'essentiel inextensibles dans toute la moitié distale (40).

2. Couche jetable pour incontinence selon la revendication 1, **caractérisée par le fait que** la zone élastique ou élastifiée respective des portions latérales arrière (22) s'étend jusqu'au bord longitudinal latéral arrière (18), mais sans enchevaucher le bord longitudinal latéral arrière.

3. Couche jetable pour incontinence selon la revendication 1 ou 2, **caractérisée par le fait que** les deux portions latérales arrière (22) sont jointes de manière inamovible à la partie principale (4) par une zone inextensible (24) enchevauchant la partie principale (4).

4. Couche jetable pour incontinence selon la revendication 1, 2 ou 3, **caractérisée par le fait que** la zone élastique ou élastifiée (42) respective est extensible dans la direction transversale (10) de 70 % au moins, en particulier de 80 % au moins, encore en particulier de 90 % au moins.

5. Couche jetable pour incontinence selon l'une ou plusieurs des revendications précédentes, **caractérisée par le fait que** l'extension (Q) des portions latérales arrière (22) dans la direction transversale (10) au-delà du bord longitudinal latéral arrière (18) respectif ainsi qu'une extension (B) maximale des portions latérales arrière (22) dans la direction longitudinale (8) est dimensionnée de telle sorte que le rapport des extensions (Q/B) l'une par rapport à l'autre est de 1,0 < Q/B < 2,0.

6. Couche jetable pour incontinence selon l'une ou plusieurs des revendications précédentes, **caractérisée par le fait que** les deux portions latérales arrière (22) présentent dans la direction longitudinale (8) une distance (d) par rapport à un bord transversal arrière (35) de la partie principale (4) qui est de 1 mm au moins, en particulier de 5 mm au moins, en particulier de 10 mm au moins, en particulier de 15 mm au moins, en particulier de 50 mm tout au plus.

7. Couche jetable pour incontinence selon l'une ou plusieurs des revendications précédentes, **caractérisée par le fait qu'**une ligne droite (45) qui s'étend dans la direction transversale (10) et est tangente au moyen de fermeture respectif du côté montrant vers l'entrejambe coupe le corps absorbant (6).

8. Couche jetable pour incontinence selon l'une ou plusieurs des revendications précédentes, **caractérisée par le fait que** chaque portion latérale arrière (22) comprend exactement un moyen de fermeture (28).

9. Couche jetable pour incontinence selon l'une ou plusieurs des revendications précédentes, **caractérisée par le fait que** les portions latérales arrière (22) sont repliées par le fabricant sur elles-mêmes autour d'au moins deux axes de pliage de portion latérale (46, 48) qui s'étendent dans la direction longitudinale (8), et que des zones partielles (50, 52, 54) des portions latérales arrière (22) sont définies et délimitées par ces axes de pliage de portion latérale (46, 48), et qu'une zone partielle (54) qui est située à l'extérieur dans la direction transversale (10) est conçue de manière à être pour l'essentiel inextensible.

10. Couche jetable pour incontinence selon la revendication 9, **caractérisée par le fait qu'**au moins 50 % de la surface d'une zone partielle (52) contiguë vers l'intérieur à la zone partielle (54) située à l'extérieur sont conçus de manière inextensible à partir de l'axe de pliage (48) extérieur s'étendant dans la direction longitudinale.

11. Couche jetable pour incontinence selon la revendication 9 ou 10, **caractérisée par le fait qu'**un axe respectif de pliage de portion latérale (46) qui, à l'état déployé, est situé à l'intérieur, donc est adjacent au bord longitudinal latéral arrière (18) de la partie principale (4), s'étend à l'intérieur de la zone élastique ou élastifiée (42) de la portion latérale (22) en question.

12. Couche jetable pour incontinence selon la revendication 9, 10 ou 11, **caractérisée par le fait qu'**un axe respectif de pliage de portion latérale (48) qui, à l'état déployé, est situé plus à l'extérieur dans la direction transversale (10), s'étend à l'intérieur d'une zone inextensible des portions latérales arrière (22).

13. Couche jetable pour incontinence selon l'une ou plusieurs des revendications précédentes, **caractérisée par le fait que** les portions latérales arrière (22) sont repliées par le fabricant sur elles-mêmes autour d'exactement deux axes de pliage de portion latérale (46, 48) s'étendant dans la direction longitudinale (8), de manière à ce qu'exactement trois zones partielles (50, 52, 54) des portions latérales (22) soient formées, et qu'au moins 50 % de la surface de la zone partielle centrale (52) sont réalisés de manière inextensible à partir de l'axe de pliage (48) extérieur s'étendant dans la direction longitudinale (8) .

14. Couche jetable pour incontinence selon l'une ou plusieurs des revendications 9 à 13, **caractérisée par le fait qu'**une extension (A) des portions latérales arrière (22) repliées sur elles-mêmes, dans la direction transversale (10) au-delà du bord longitudinal latéral arrière (18) respectif ainsi qu'une extension (B) des portions latérales arrière (22) repliées sur elles-mêmes, dans la direction longitudinale (8), est dimensionnée de telle sorte que le rapport des extensions (A/B) l'une par rapport à l'autre est de 0,5 < A/B < 1.

15. Couche jetable pour incontinence selon l'une ou plusieurs des revendications 9 à 14, **caractérisée par le fait que** la partie principale (4) est repliée, par le fabricant, vers l'intérieur sur elle-même, conjointement avec les portions latérales arrière (22) repliées sur elles-mêmes, autour de premier et de deuxième axes de pliage de partie principale s'étendant chacun dans la direction longitudinale (8), de telle sorte que les portions latérales arrière (22) situées de part et d'autre viennent se positionner, dans la direction de l'épaisseur, de manière à se chevaucher au moins en partie l'une l'autre.
